# EUROPEAN PATENT APPLICATION

(11) **EP 4 628 593 A1**
(43) Date of publication of application: **08.10.2025**
(21) Application number: 23943153.9
(22) Date of filing: 27.09.2023
(51) Int. Cl.: C12Q 1/6811, C12Q 1/6869

(54) **METHOD FOR DETECTING METHYLATION AND MUTATION STATES OF DNA SAMPLE**

(30) Priority: 29.06.2023 CN 202310782691
(71) Applicant: Nanodigmbio (Nanjing) Biotechnology Co., Ltd, Nanjing, Jiangsu 210000 (CN)
(72) Inventor: YU, Liping, Nanjing, Jiangsu 210000 (CN); WANG, Biao, Nanjing, Jiangsu 210000 (CN); WU, Qiang, Nanjing, Jiangsu 210000 (CN)
(74) Representative: Novagraaf Technologies
(86) International application number: PCT/CN2023/114356
(87) International publication number: WO 2025/000669

(57) **Abstract**

Provided herein is a method for detecting methylation and mutation states of a DNA sample, and particularly, provided is a method for simultaneously detecting a methylation state and a mutation state of a DNA molecule in a sample. The method includes treating a sample with a methylation-sensitive restriction endonuclease; using a probe group including a mutation capture probe and a methylation capture probe to carry out hybrid capture on amplification products of the sample treated with the restriction endonuclease and a sample untreated with enzyme digestion; and sequencing a capture product.

## Description

The present application claims priority to Chinese Patent Application No. 2023107826914, filed with the China National Intellectual Property Administration on June 29, 2023, the entire contents of which are hereby incorporated by reference.

### Technical Field

The present application relates to a method for simultaneously detecting methylation and mutation states, where methylation and/or mutation detection of a target region is completed by means of capture.

### Background

According to statistics, there were approximately 4.82 million new cancer cases and 3.21 million cancer death cases in China in 2022. The cancer has become a globally recognized public health issue. Early screening and early diagnosis of cancers is a critical pathway to reducing a cancer mortality. According to a report published by Grand View Research, Inc. on multi-cancer early detection, the global multi-cancer early detection market size is expected to reach USD 2.77 billion by 2030, expanding at a compound annual growth rate of 15.80% from 2022 to 2030. The rise of multi-cancer early detection technologies has epoch-making significance for early screening and early diagnosis of cancers. Compared with single-cancer detection, multi-cancer detection can achieve the detection of multiple cancers with a single detection and has become the future trend of development in the industry. With the development of the early screening industry of cancers and the expansion of market size, an increasing number of enterprises are now focusing on the field of early screening of cancers, achieving the crossing from the single-cancer detection to the multi-cancer detection.

Clinical early tumor screening methods include imageology, endoscopy, and tumor markers. These detection technologies have defects such as invasiveness, screening discomfort, and low detection sensitivity. The conventional methods have various limitations. There is an urgent clinical need for some early screening technologies with high sensitivity/specificity and convenient operation. Liquid biopsy, as a novel non-invasive detection technology, has immense application potential for early screening of tumors. Tumor markers for liquid biopsy mainly include levels such as genomics, epigenetics, transcriptomics, microbiomics, proteomics, and metabolomics. The characteristics of the tumor markers at a ctDNA level include dimensions such as point mutation, fragmentation, copy number variation, and methylation. Mutation is the most proficient technology for gene detection companies. However, early cancer mutation signals are weak and highly difficult to detect, and the mutation signals are susceptible to interference from clonal hematopoiesis signals, facing sore points such as the lack of tumor specificity and the difficulty in tracing tumor positions of origin. Methylation markers, characterized by stable presence in plasma, abundance of variation sites, capability for tissue of origin tracing, etc., have become preferred markers in the field of early screening of cancers. The occurrence and development of cancers involve changes at multiple levels, and single-level assessments have certain limitations. Therefore, "multi-omics" has become one of the hot technical terms in the field of tumor detection in early screening of cancers. The combination of mutation and methylation detection technologies enables advantage complementation, facilitating the implementation of multi-cancer early screening technologies.

There are various methylation sequencing methods. First-generation and second-generation sequencing technologies can only distinguish the four bases A, T, C, and G, but cannot distinguish methylated and non-methylated C bases. The conventional treatment scheme is to use bisulfite to convert an unmethylated C base to a U base, convert the U base to T through PCR amplification, and compare with an unconverted sequence to determine whether a methylation modification occurs at the site. However, since the converted sequence differs from the original sequence, the liquid phase hybrid capture scheme based on existing systems cannot achieve the experimental objective of the co-detection of mutation and methylation in the same hybrid capture system, resulting in that methylation detection and mutation detection cannot be carried out simultaneously.

### Summary

Provided herein is a method for simultaneously detecting a methylation state and a mutation state of a DNA molecule in a sample, including:
1) adding adapter molecules to both ends of the DNA molecule in the sample to obtain an adapter ligation product sample; and dividing the adapter ligation product sample into two parts: a first adapter ligation product sample and a second adapter ligation product sample;
2) treating the first adapter ligation product sample with one or more methylation-sensitive restriction endonucleases, such that the DNA molecule is capable of being subjected to an enzyme digestion reaction when methylation is not present at a recognition site of the methylation-sensitive restriction endonuclease, thereby obtaining an enzyme digestion sample; and untreating the second adapter ligation product sample with the restriction endonuclease;
3) amplifying the enzyme digestion sample and the second adapter ligation product sample respectively with a primer specific to the adaptor molecule and having an Index sequence, to respectively obtain an enzyme digestion sample amplification product and a second adapter ligation product sample amplification product; and mixing the enzyme digestion sample amplification product with the second adapter ligation product sample amplification product to obtain an amplification product mixture;
4) making a probe group including one or more mutation capture probes and one or more methylation capture probes in contact with the amplification product mixture to obtain a capture product, wherein the methylation capture probe targets a target sequence including the recognition site in the DNA molecule, and the mutation capture probe targets a target sequence including a mutation site in the DNA molecule; and
5) sequencing the capture product; determining the methylation state of the DNA molecule including the recognition site by means of a sequencing depth ratio of recognition sites from the enzyme digestion sample amplification product and the second adapter ligation product sample amplification product; and determining the mutation state by means of a sequencing result of the mutation site.

In some embodiments, the mutation capture probe and/or the methylation capture probe include a target specific sequence, a first probe binding sequence located at a 5' end of the target specific sequence, and a second probe binding sequence located at a 3' end of the target specific sequence; and the first probe binding sequence is at least partially complementary to the second probe binding sequence, such that when two or more of the mutation capture probes and/or two or more of the methylation capture probes bind to a target sequence thereof adjacently, the adjacent mutation capture probes and/or the adjacent methylation capture probes are capable of being complementarily bound by means of the first probe binding sequence and the second probe binding sequence.

In some embodiments, the probe group further includes one or more normalization probes.

In some embodiments, the first probe binding sequence and the second probe binding sequence have a length of 8-30 nt.

In some embodiments, the target specific sequence has a length of 20-80 nt.

In some embodiments, the method, prior to step 1), further includes, fragmenting the DNA molecule.

In some embodiments, the methylation-sensitive restriction endonuclease is selected from Hpa I, Hpa II, Hha I, Aci I, and any combination thereof.

In some embodiments, the sample includes a ctDNA.

In some embodiments, the methylation state includes whether methylation is present and/or a methylation level.

In some embodiments, the mutation state includes whether mutation is present and/or a mutation type.

In some embodiments, the mutation type is selected from base insertion, deletion and substitution, chromosomal copy number variation, microsatellite instability, and gene fusion.

### Brief Description of the Drawings

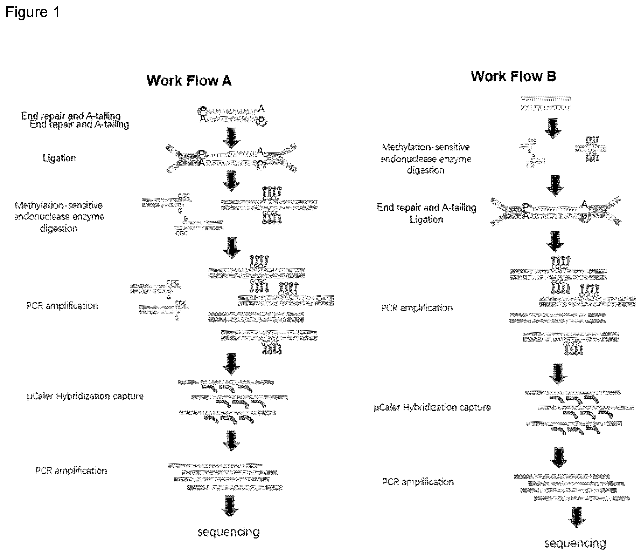

Fig. 1 shows a process for preparing a capture library.

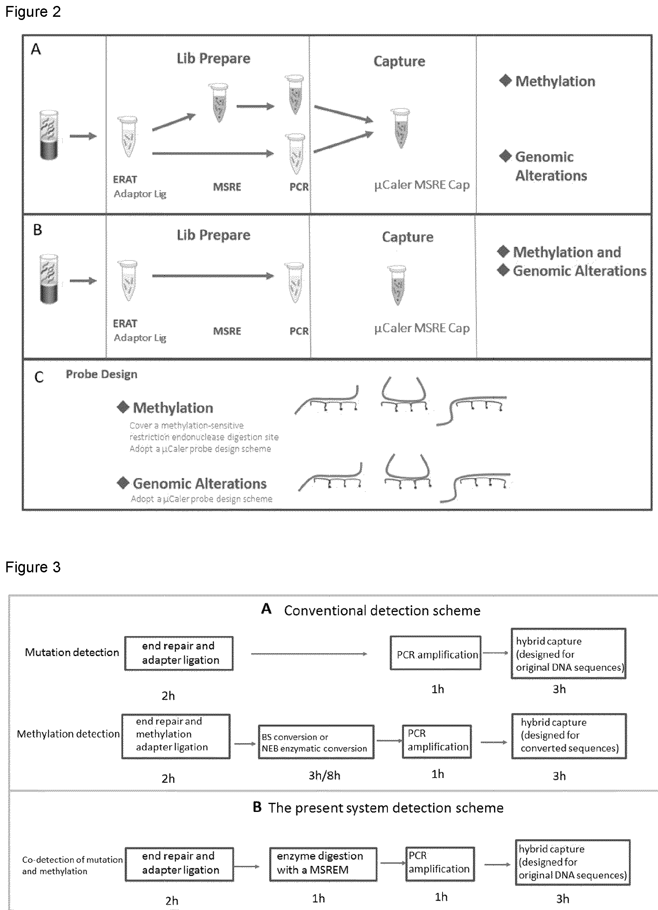

Fig. 2 shows an experimental process of co-detection of capture and mutation and a corresponding probe design scheme. (A): Start an experiment according to the experimental process described in Fig. 1; after adapter product ligation, use a methylation-sensitive restriction endonuclease for enzyme digestion, libraries prepared from enzyme digestion products being used for detection of a methylation state, and libraries without enzyme digestion being used for mutation detection. (B): Start an experiment according to the experimental process described in Fig. 1; after adapter product ligation, use a methylation-sensitive restriction endonuclease for enzyme digestion, libraries prepared from enzyme digestion products being used for analysis of a methylation state and a mutation detection state. (C): A thought for probe design: carry out methylation detection, with a probe needing to cover a methylation-sensitive restriction endonuclease digestion site; carry out mutation site detection, with a probe covering a genetic site to be detected. If the experimental process in B is adopted, the design of a mutation detection probe needs to consider methylation detection, and the designed probe cannot cover a methylation-sensitive restriction endonuclease digestion site.
Fig. 3 shows a comparison of experimental processes of mutation and methylation detection between a conventional detection scheme and a scheme adopted in the present application. (A): A process for mutation and methylation detection of a conventional detection scheme, where two sets of libraries need to be prepared, and two groups of hybrid capture experiments need to be carried out. A methylation library uses a methylation-modified adapter, with a relatively high adapter cost. An adapter ligation product is treated with a BS or EM conversion module to convert a non-methylation-modified C base to a U base. (B): A scheme of co-detection of mutation and methylation of the present application, where a common DNA adapter is used; MSRE is used for enzyme digestion; methylation-modified and non-methylation-modified C bases are distinguished; this system prepares one DNA library, or an adapter ligation product is divided into two parts: one is subjected to enzymatic digestion and then amplified, and the other is directly amplified; both the amplification products can be hybridized in the same hybrid capture system.

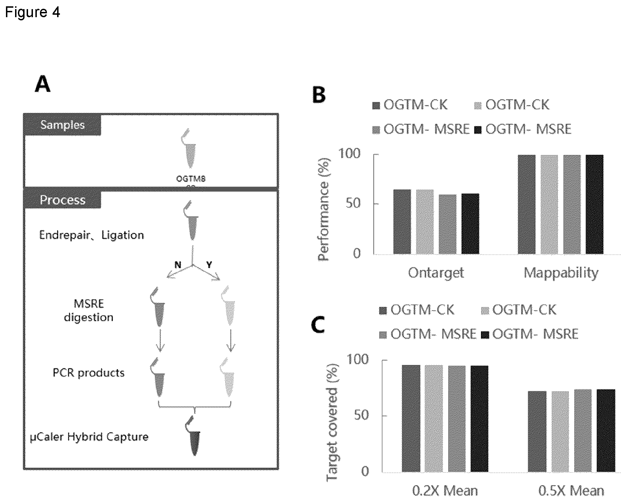

Fig. 4 shows an experimental process and results of post-run quality control. (A) Experimental process; (B) Performance results: On-Target, representing Fraction of Target Reads in mapped reads; Mappability, representing the rate of data mapped into a genome; (C) 0.5× Mean Coverage, representing the percentage of 0.5× mean coverage; 0.2× Mean Coverage, representing the percentage of 0.2× mean coverage.

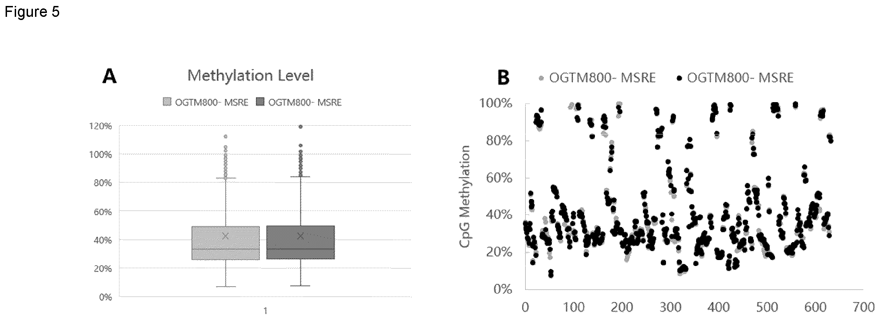

Fig. 5 shows sequencing depths and corresponding methylation levels of CpG sites associated with a methylation-sensitive restriction endonuclease within target regions of different libraries, where a methylation level calculation method: enzyme digestion depth/control depth; (A) Mean CpG methylation level of two enzyme digestion experimental groups; (B) Methylation level corresponding to each CpG site.

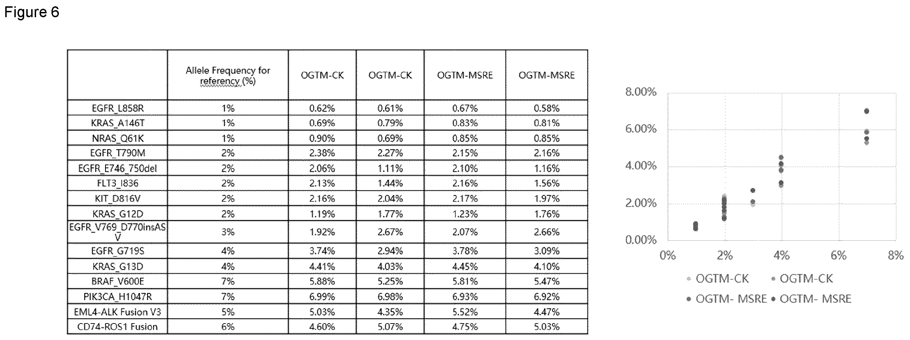

Fig. 6 shows results of consistency analysis between mutation detection frequencies and theoretical mutation frequencies in capture data.

### Detailed Description of the Embodiments

Unless specified otherwise, all technical and scientific terms used herein have the meanings generally understood by those of ordinary skill in the art.

The term "or" refers to a single element from the enumerated selectable elements unless the context explicitly indicates otherwise. The term "and/or" refers to any one, any two, any three, any more, or all of the enumerated selectable elements.

The terms "comprising", "including", "having", and similar expressions used herein represent not excluding elements that are not enumerated. These terms also include instances consisting solely of the enumerated elements.

"DNA molecule" herein refers to deoxyribonucleic acid, which is a polymer of deoxyribonucleotides. It may be in a double-stranded form or may be in a single-stranded form, or, for a population of DNA molecules, may include a double-stranded DNA molecule or may include a single-stranded DNA molecule. The DNA molecule may be of any length, e.g., may be genomic DNA, a DNA fragment, or extracellular free DNA. Free DNA refers to DNA that is free outside the cell or the cell nucleus, and can be extracted from biological materials such as various body fluids, *in-vitro* cell culture fluids, and natural environments, including but not limited to: peripheral blood, plasma, serum, urine, feces, saliva, cerebrospinal fluid, and lymphatic fluid. The free DNA can be obtained through extraction and purification. "DNA fragment" herein refers to a shorter DNA molecule, e.g., with a length of 50 bp to 700 bp, e.g., 100 bp to 500 bp, particularly 100 bp to 350 bp. DNA fragments included in a biological sample are generally heterogeneous, i.e., they vary in length. Accordingly, the aforementioned lengths may refer to the mean length of these DNA fragments. These DNA fragments may have different sequences, e.g., derived from different regions of the same organism's genome or even derived from different organisms. The DNA fragments may have single-strand nicks, or may have blunt ends or non-blunt ends (with 3' overhangs or 5' overhangs).

"DNA methylation" herein refers to the methylated modification of bases in DNA molecules or DNA fragments, particularly the modification of cytosine into 5-methylcytosine (5mC). The DNA methylation in vertebrates generally occurs at a CpG site (i.e., the site that follows cytosine and is closely linked to guanine in a DNA sequence), where cytosine is converted to 5-methylcytosine through the catalysis of a DNA methyltransferase. Most CpG sites in the human genome are already methylated. However, in certain specific regions, e.g., CpG islands enriched with cytosine (C) and guanine (G), they are generally unmethylated. CpG methylation can affect the transcriptional activity of associated genes. For example, methylation can suppress cancer suppressor genes, while demethylation can stimulate the expression of certain oncogenes, both of which may cause the occurrence of cancers. Additionally, although the occurrence rate is lower than that of 5mC, a small number of cytosine bases are modified into 5-hydroxymethylcytosine (5hmC), 5-formylcytosine (5fC), and 5-carboxylcytosine (5caC). References herein to methylation may also refer to modifications into 5hmC, 5fC, and 5caC, unless the context indicates otherwise.

"Cytosine base methylation state" or "DNA methylation information" herein refers to information about the methylation condition in a DNA molecule or a DNA fragment, including but not limited to methylation sites, methylation levels, and methylation modes (e.g., 5mC or 5hmC). "Methylation level", also referred to as "methylation degree", refers to the proportion (or frequency) of methylated modification of a specific methylation site in a sample. Various methods can be used for detecting whether a site is methylated. Common methods include chemical or enzymatic conversion methods, where either methylated cytosine or unmethylated cytosine is converted to uracil (U) or a base (e.g., dihydrouracil, DHU) basically equivalent to uracil in base pairing, during conversion. In the subsequent amplification process, the corresponding uracil as thymine (T) pairs with adenine (A), finally resulting in that the cytosine or methylated cytosine at the methylation site is represented as thymine in the detection results (e.g., sequencing results). By comparing with a reference sequence, it can be determined whether the cytosine in the DNA molecule or the DNA fragment is methylated. The reference sequence may be a sequence that is derived from the same sample without undergoing the aforementioned conversion or may be a corresponding sequence in healthy populations. 5mC and 5hmC can also be distinguished by some methods. Currently, DNA methylation information has been widely applied in screening and diagnosis of cancers (e.g., lung cancer, breast cancer, liver cancer, and colorectal cancer), particularly in early screening and diagnosis. Additionally, the identification of methylation state can also be used for non-diagnostic (or non-therapeutic) purposes, e.g., used for scientific research (e.g., analyzing factors influencing methylation, and the impact of methylation on gene function), or used for the detection of methylation standards.

"Methylation-sensitive restriction endonuclease" refers to a restriction endonuclease that is sensitive to whether its recognition site includes a methylated base. Such enzymes generally cannot cleave DNA molecules at their recognition sites if their recognition sites include a methylated base. The methylation-sensitive restriction endonuclease herein specifically refers to a restriction endonuclease that cannot cleave a DNA molecule at its recognition site when the recognition site includes at least one 5mC. Methylation-sensitive restriction endonucleases known in the art include, but are not limited to, Hpa I, Hpa II, Hha I, and Aci I.

"Capture probe" herein refers to a single-stranded DNA fragment used for hybridizing with a target DNA molecule. The capture probes herein include a methylation capture probe and a mutation capture probe. The methylation capture probe used preferably targets a target sequence including a restriction endonuclease recognition site in a DNA molecule. When a DNA molecule is treated with a specific restriction endonuclease, if the methylation state of its recognition site enables the restriction endonuclease to cleave the DNA molecule, the capture probe can only partially or insufficiently bind to the target DNA molecule, thereby preventing effective isolation of a restriction endonuclease-cleaved target DNA molecule from the sample. Under such conditions, the target DNA molecules that can be isolated from samples treated with a restriction endonuclease by using the methylation capture probe are mainly DNA molecules that are uncleaved by the restriction endonuclease. Consequently, the overall methylation state of DNA molecules in the sample can be determined according to the quantity of uncleaved DNA molecules that can be isolated relative to the quantity of DNA molecules that can be isolated from samples untreated with the restriction endonuclease. For example, when treated with a methylation-sensitive restriction endonuclease, the quantity of cleaved DNA molecules is relatively high (the quantity of uncleaved DNA molecules is relatively low), indicating that the DNA molecules in the sample have a lower methylation level at the restriction endonuclease recognition site. In a preferred embodiment, the methylation capture probe is a µCaler probe (for the design of µCaler probe, refer to Chinese Patent Publication CN116083423A, the entire contents of which are hereby incorporated by reference). The probe includes a target specific sequence, a first probe binding sequence located at a 5' end of the target specific sequence, and a second probe binding sequence located at a 3' end of the target specific sequence. The first probe binding sequence is at least partially complementary to the second probe binding sequence, such that when the two or more capture probes bind to the target sequence adjacently, the adjacent capture probes are capable of being complementarily bound by means of the first probe binding sequence and the second probe binding sequence. Compared with common capture probes, the advantages of using the µCaler probe lie in significantly improving the capacity of a probe binding to a target, improving the hybrid capture efficiency of a target region, and improving the overall coverage uniformity and stability. The capture probe generally has an isolatable tag, e.g., biotin, to facilitate the isolation of target DNA molecules binding to the probe. The sequence information of different target DNA molecules isolated can be obtained by means of sequencing, and the methylation state at the restriction endonuclease recognition site can also be obtained. The mutation capture probe is a capture probe that targets a mutation site of interest included in a DNA molecule, and the information about the mutation state of a specific mutation site can be obtained by sequencing DNA molecules captured by the mutation capture probe. Preferably, the mutation capture probe is also a µCaler probe. The methylation capture probe and the mutation capture probe may be located in the same probe group, achieving the simultaneous capture of respective target sequences. Additionally, the probe group used herein may also include a normalization probe in order to normalize the detection results between different samples. Preferably, the normalization probe is also a µCaler probe.

In some embodiments, provided herein is a process for preparing a targeted methylation library based on a methylation-sensitive restriction endonuclease, and the methylation-sensitive restriction endonuclease includes Hpa I, Hpa II, Hha I, and Aci I.

In some embodiments, the targeted capture system provided herein is for non-converted library capture; original DNA sequences are captured; methylation modification occurs at all CpG sites of restriction endonuclease recognition sites in captured fragment target regions; the information of methylation modification occurring in the target regions is detected.

In some embodiments, the method provided herein is used for mutation detection in a DNA sample, and the mutation types include: base substitution, chromosomal copy number variation analysis, insertion/deletion, microsatellite instability, or gene fusion.

In some embodiments, the capture probes used are designed for original DNA strands and detect methylation-related sites, and a targeted capture probe needs to cover a corresponding methylation-sensitive restriction endonuclease digestion site.

In some embodiments, two different libraries are used respectively for co-detection of mutation and methylation, and the design of the mutation capture probe does not need to consider the design of the methylation capture probe. In some embodiments, the same library is used for co-detection of mutation and methylation, and the mutation capture probe cannot cover a methylation-sensitive restriction endonuclease recognition sequence associated with methylation detection.

In some embodiments, provided herein is a process for preparing a targeted methylation library based on a methylation-sensitive restriction endonuclease; the sequencing depth of CpG sites can be detected by means of the process; the methylation degree of CpG sites in the sample is analyzed by means of control.

In some embodiments, the method provided herein employs a molecular tag-containing adapter module, and a molecular tag filters background noise and is suitable for detecting a low-frequency mutation signal.

For the process for preparing a targeted capture library herein, refer to Fig. 1. The process is suitable for detection of methylation states and mutation signals in target regions of a physically disrupted gDNA sample and a plasma free DNA or circulating tumor ctDNA sample. Fragmented samples are for library preparation, and a kit for library preparation is Nadprep Universal DNA Library Preparation Kit. The process for library preparation is divided into two schemes: Workflow A: End repair and A-tailing, and adapter ligation. An adapter ligation product is subjected to enzyme digestion with a methylation-sensitive restriction endonuclease. The types of the restriction endonuclease include Hpa I, Hpa II, Hha I, and Aci I. The other group is not subjected to enzyme digestion to serve as a control group. The two groups of products are respectively subjected to PCR amplification, with the addition of an Index sequence. The amplification products are subjected to hybrid capture with a µCaler hybrid Capture system. All probes adopt the µCaler probe design scheme. The probes are designed for original DNA sequences. The probes cover a methylation-sensitive restriction endonuclease digestion site for the detection of a methylation state and cover a mutation detection site for the detection of a mutation signal. Workflow B: The methylation-sensitive restriction endonuclease is used for enzyme digestion. The types of the restriction endonuclease include Hpa I, Hpa II, Hha I, and Aci I. The enzyme digestion products are subjected to purification and recovery. Then library preparation is carried out. The library preparation products are subjected to hybrid capture with a µCaler hybrid Capture system. All probes adopt the µCaler probe design scheme. The probes are designed for original DNA sequences. The probes cover a methylation-sensitive restriction endonuclease digestion site for the detection of a methylation state and cover a mutation detection site for the detection of a mutation signal. Captured libraries are sent to an Illumina or MGI platform sequencer for sequencing.

For the experimental process of co-detection of capture and mutation and the corresponding experimental design scheme, refer to Fig. 2. A: Start an experiment according to the experimental process described in Fig. 1; after adapter product ligation, use a methylation-sensitive restriction endonuclease for enzyme digestion, libraries prepared from enzyme digestion products being used for detection of a methylation state, and libraries without enzyme digestion being used for mutation detection. B: Start an experiment according to the experimental process described in Fig. 1; after adapter product ligation, use a methylation-sensitive restriction endonuclease for enzyme digestion, libraries prepared from enzyme digestion products being used for analysis of a methylation state and a mutation detection state. C: A thought for probe design of this system: carry out methylation detection, with a probe needing to cover a methylation-sensitive restriction endonuclease digestion site; carry out mutation site detection, with a probe covering a genetic site to be detected. If the experimental process in B is adopted, the design of a mutation detection probe needs to consider methylation detection, and the designed probe cannot cover a methylation-sensitive restriction endonuclease digestion site.

Adopting this system for the process of co-detection of mutation and methylation has significant advantages compared with a conventional scheme, as detailed in Fig. 3, which shows the comparison of experimental processes of mutation and methylation detection between a conventional detection scheme and a scheme adopted in this system. In the process of mutation and methylation detection of the conventional detection scheme (Fig. 3A), two sets of libraries need to be prepared, and two groups of hybrid capture experiments need to be carried out. A methylation library uses a methylation-modified adapter, with a relatively high adapter cost. An adapter ligation product is treated with a BS (experimental time of 2 hours) or EM (experimental time of 8 hours) conversion module, wherein a BS conversion process is relatively cumbersome and has a relatively severe conversion damage, so that using this scheme results in a substantial loss of original sample information; the EM conversion module has a relatively low conversion damage, and it is recommended to use an NEBNext Enzymatic Conversion module to convert an unmethylated C base to a U base and use an amplification enzyme (KAPA HiFi HotStart Uracil Mix), capable of recognizing the U base, to carry out PCR amplification. A mutation detection library uses a common adapter module. Considering that the converted library DNA sequences are altered, a probe used during hybrid capture is designed for the converted DNA sequences. The methylation library and the DNA library must use independent hybrid capture systems to carry out experiments. In the scheme (Fig. 3B) of co-detection of mutation and methylation of this system, a common DNA adapter is used; MSRE is used for enzyme digestion (experimental time of 1 hour); methylation-modified and non-methylation-modified C bases are distinguished; this system prepares one DNA library, or an adapter ligation product is divided into two parts: one is subjected to enzymatic digestion and then amplified, and the other is directly amplified; both the amplification products can be hybridized in the same hybrid capture system. Adopting the conventional scheme for mutation and methylation detection may take time of 2-3 days, whereas using the scheme of this system may shorten the experimental time to one day, so that this system greatly shortens wet lab time, simplifies the experimental process, and saves reagent costs, demonstrating significant advantages.

A methylation level analysis scheme: for samples subjected to enzyme digestion, samples not subjected to enzyme digestion of different methylation levels are respectively used as controls to calculate the methylation levels of different samples. A methylation level calculation method: each site is normalized with an internal reference gene, i.e., the normalized depth of each CpG site of the samples subjected to enzyme digestion/the normalized depth of each CpG site of the samples in the control group.

A mutation detection analysis scheme: Vardict or GATK software is used for variant calling. When the Vardict software is used for analysis, Hg19 is used as a bed file to display coordinates of hg19. When the GATK software is used for analysis, the control samples are used for variant calling.

The present application aims to develop a technical scheme that completes co-detection of mutation and methylation in the same hybrid capture system, which shortens detection time, simplifies the experimental process, increases the sample utilization rate, reduces reagent costs, and has broad market application prospects, compared with conventional detection technical schemes.

### General experimental steps:

### Step 1: Sample fragmentation

DNA sample fragmentation could be carried out according to laboratory conditions. It was recommended to use a Covaris^{™} series DNA ultrasonicator to fragment the samples into products with a mean fragment length of 200-250 bp. gDNA or FFPE DNA proceeded to Step 2 after disruption. cfDNA samples proceeded directly to Step 2.

### Step 2: End Repair & A-Tailing

1. End Repair & A-Tailing Buffer was taken for thawing at room temperature, mixed thoroughly, and placed on ice for standby use.
2. End Repair & A-Tailing Enzyme was taken to be placed on ice for natural thawing, mixed thoroughly, and instantaneously centrifuged for standby use.
3. A reaction system was prepared in a 0.2 mL PCR tube on ice, according to the table below:

| | |
|---|---|
| Fragmented gDNA or cfDNA | 20 µL |
| End Repair & A-Tailing Buffer | 3 µL |
| End Repair & A-Tailing Enzyme | 2 µL |
| Total | 25 µL |

| | |
|---|---|
| Note: If the DNA volume is less than 20 µL, supplement Nuclease Free Water to 20 µL. | |

4. Thorough mixing and instantaneous centrifugation made all reaction liquids be at the bottom of the PCR tube.
5. The following reaction programs (Cycling Program I) were initiated on a PCR instrument. When the temperature stabilized to 20°C, the PCR tube was placed into the PCR instrument:

| | |
|---|---|
| 20 °C | 30 min |
| 65 °C | 30 min |
| 10 °C | Hold |

| | |
|---|---|
| Note: Do not use a thermal lid during 20°C program running, and set the thermal lid to 70°C during 65°C program running. | |

### Step 3: Adapter ligation

1. Ligation Buffer was taken for thawing at room temperature, mixed thoroughly, and placed on ice for standby use.

Note: the Ligation Buffer is highly viscous, pipetted and released slowly and steadily by a pipettor to ensure an accurate volume.
2. DNA Ligase was taken to be placed on ice for natural thawing, mixed thoroughly, and instantaneously centrifuged for standby use.
3. The PCR tube in Step 2 was taken out from the PCR instrument and placed on ice plates. A reaction system was prepared according to the table below:

| | |
|---|---|
| Reaction product in Step 2 | 25 µL |
| NadPrep^{®} Universal Stubby Adapter | 2 µL |
| Ligation Buffer | 13 µL |
| DNA Ligase | 2 µL |
| Total | 42 µL |

### Step 4: Purification of ligation product

Purification and recovery were carried out with 0.5× Beads. The purification process was as follows:
1. 20 µL of NadPrep^{®} SP Beads were added to the ligation reaction product in Step 3. Thorough mixing was carried out. Incubation was carried out at 20-25°C for 10 minutes.
2. The PCR tube was instantaneously centrifuged and then placed on a magnetic rack for 5 minutes until the liquid was completely clear. The supernatant was pipetted with a pipettor and discarded.
   Note: the liquid must be completely clear. Different brands of magnetic racks require the adjustment of placement time.
3. 150 µL of 80% ethanol was slowly added along the sidewall of the PCR tube. Note: Do not disturb magnetic beads. Being standing was carried out for 30 seconds. The supernatant was pipetted with a pipettor and discarded.
4. Step 3 was repeated once.
5. The PCR tube was instantaneously centrifuged and then placed on a magnetic rack. A small amount of residual ethanol was removed with a 10 µL tip. Note: Do not pipette the magnetic beads.
6. The PCR tube lid was opened. Being standing was carried out at 20-25°C for about 2-3 minutes until the ethanol volatilized completely.
7. The PCR tube was transferred. 44 µL of Nuclease Free Water was added to the PCR tubes. The magnetic beads were resuspended thoroughly with a pipettor. Incubation was carried out at 25°C for 2 minutes.
8. The PCR tube was instantaneously centrifuged and then placed on a magnetic rack for 2 minutes until the liquid was completely clear. The supernatant was carefully transferred with a pipettor to a new 0.2 mL PCR tube.

### Step 5: Enzyme digestion with methylation-sensitive restriction endonuclease

1. MeEnzyme & MeBuffer were taken to be placed on ice for natural thawing (NEB restriction endonuclease), mixed thoroughly, and instantaneously centrifuged for standby use.
2. A reaction system was prepared in a 0.2 mL PCR tube on ice, according to the table below. Using a methylation-sensitive restriction endonuclease, the system was as follows:

| | |
|---|---|
| MeEnzyme (methylation-sensitive restriction endonuclease) | 1 µL |
| MeBuffer | 5 µL |
| Ligation product purified in Step 4 | 44 µL |
| Total | 50 µL |

3. The following reaction programs were initiated on a PCR instrument. When the temperature stabilized to 37°C, the PCR tube was placed into the PCR instrument: 37°C for 1 hour; 80°C for 20 minutes.

### Step 6: Purification of enzyme digestion product

Purification and recovery were carried out with 1 × Beads. For the purification process, refer to Step 4. Elution was carried out with 20 µL of Nuclease Free Water.

### Step 7: PCR amplification of enzyme digestion product

1. 2× HiFi PCR Master Mix and NadPrep^{®} Universal UDI-Index Primer Mix were taken to be placed on ice for natural thawing, mixed thoroughly, and instantaneously centrifuged.
2. PCR amplification Mix was prepared in a 0.2 mL PCR tube on ice, according to the table below:

| | |
|---|---|
| Enzyme digestion product purified in Step 6 | 20 µL |
| NadPrep^{®} Universal UDI-Index Primer Mix | 5 µL |
| 2 × HiFi PCR Master Mix | 25 µL |
| Total | 50 µL |

3. The PCR tube was placed into the PCR instrument and the following programs were initiated:

| | | |
|---|---|---|
| 98 °C | 2 min | |
| 98 °C | 15 s | |
| 60 °C | 30 s | 6-8 cycles |
| 72 °C | 30 s | |
| 72 °C | 2 min | |
| 4 °C | Hold | |

### Step 8: Purification of amplification product

Purification and recovery were carried out with 1 × Beads. For the purification process, refer to Step 4. Elution was carried out with 20 µL of Nuclease Free Water.

### Step 9: Library hybridization

1. A hybrid reaction system was prepared according to the table below:

| | |
|---|---|
| Sample | 18 µL |
| uCaler NanoBlockers | 2 µL |
| uCaler ReMe Panel. uCaler Mutation Panel | 2 µL |
| µHyb #1 | 30 µL |
| µHyb #2 | 6 µL |

2. The hybrid reaction mixed liquid was vortexed and mixed thoroughly for more than 10 seconds. After instantaneous centrifugation, the reaction mixed liquid was collected to the bottom of the PCR tube (avoiding bubbles).
3. The PCR tube was placed into the PCR instrument and the following reaction programs were initiated: 98°C for 2 minutes; 60°C for 1 hour.

### Step 10: Library capture and elution

### Magnetic bead washing

1. Streptavidin Beads were vortexed for 15 seconds to ensure thorough mixing.
2. n× 25µL Streptavidin Beads were taken and subjected to mixed washing in a 0.2 mL centrifugal tube (n was the number of captured libraries, with n < 5).
   Note: When n > 5, mixed washing needs to be carried out in multiple tubes.
3. The Streptavidin Beads were placed on a magnetic rack to be standing for about 2 minutes until the liquid was completely clear. The supernatant was discarded with a pipettor.
4. The centrifugal tube was transferred from the magnetic rack. 100 µL of pre-heated Wash Buffer A was added. Thorough mixing gently by pipetting was carried out more than 10 times.
5. The centrifugal tube was placed on the magnetic rack to be standing for about 2 minutes until the liquid was completely clear. The supernatant was discarded with a pipettor.
6. Step 4 and Step 5 were repeated once.
7. The centrifugal tube was instantaneously centrifuged. All the Wash Buffer A at the tube bottom was removed with a 10 µL tip. 8. n× 8 µL uHyb#I was taken to resuspend the Streptavidin Beads. Thorough mixing gently by pipetting was carried out more than 10 times.

### Magnetic Bead Capture

1. After the hybrid reaction for 1 hour, the step proceeded to the capture link. The PCR instrument was kept in a running state.
2. In a state where the PCR tube was placed in the PCR instrument, 8 µL of resuspended Streptavidin Beads were taken to be immediately added to each hybrid reaction liquid. Thorough mixing gently by pipetting was carried out more than 10 times.
3. Incubation was carried out at 60°C for 10 minutes.
4. After incubation, the PCR tube was removed from the PCR instrument to be placed on the magnetic rack to be standing for 2 minutes until the liquid was completely clear. The supernatant was discarded with a pipettor (the supernatant was removed as completely as possible).

### Elution

1. The PCR tube in the previous step was transferred from the magnetic rack. 150 µL of pre-heated Wash Buffer A was added. Thorough mixing gently by pipetting was carried out more than 10 times (avoiding bubbles).
2. The PCR tube was placed on the magnetic rack to be standing for 2 minutes until the liquid was completely clear. The supernatant was discarded with a pipettor. 3. The PCR tube was transferred from the magnetic rack. 100 µL of pre-heated Wash Buffer A was added. Thorough mixing gently by pipetting was carried out more than 10 times. The reaction liquid was transferred to a new PCR tube.
   Note: bubbles are avoided as much as possible in this step, bubbles contacting the tube lid may result in a reduced on-target rate.
3. The PCR tube was placed into the PCR instrument. Incubation was carried out at 60°C for 3 minutes.
4. The PCR tube was placed into the PCR instrument. Incubation was carried out at 60°C for 3 minutes.
5. After incubation, the PCR tube was removed from the PCR instrument to be placed on the magnetic rack to be standing for 2 minutes until the liquid was completely clear. The supernatant was discarded with a pipettor.
6. 150 µL of Wash Buffer B placed at room temperature was added to the PCR tube. Thorough mixing gently by pipetting was carried out more than 10 times.
7. The PCR tube was placed on the magnetic rack to be standing for 2 minutes until the liquid was completely clear. The supernatant was discarded with a pipettor.
8. The PCR tube was instantaneously centrifuged and then placed on the magnetic rack. A small amount of residual liquid was removed with a 10 µL tip. Note: Do not pipette the magnetic beads.
9. 22.5 µL of Nuclease Free Water was added. Washing gently by pipetting was carried out more than 10 times to resuspend the magnetic beads.

### Step 11: PCR amplification of hybrid capture library

1. 2× HiFi PCR Master Mix and NadPrep^{®} Universal UDI-Index Primer Mix were taken to be placed on ice for natural thawing, mixed thoroughly, and instantaneously centrifuged.
2. PCR amplification Mix was prepared in a 0.2 mL PCR tube on ice, according to the table below:

| | |
|---|---|
| Enzyme digestion product purified in Step 6 | 20 µL |
| NadPrep^{®} Primer Mix | 5 µL |
| 2 × HiFi PCR Master Mix | 25 µL |
| Total | 50 µL |

3. The PCR tube was placed into the PCR instrument and the following programs were initiated:

| | | |
|---|---|---|
| 98 °C | 2 min | |
| 98 °C | 15 s | |
| 60 °C | 30 s | 10 cycles |
| 72 °C | 30 s | |
| 72 °C | 2 min | |
| 4 °C | Hold | |

### Step 12: Purification of amplification product

Purification and recovery were carried out with 1 × Beads. For the purification process, refer to Step 4. Elution was carried out with 20 µL of Nuclease Free Water.

### Example: Co-detection of Mutation and Methylation by Using Standard Samples

OGTM800 standard samples were used for preparing two libraries. The process for library preparation is briefly described as follows: 40 ng of an ultrasonically disrupted standard sample was taken for end repair and A-tailing, and adapter ligation; the adapter ligation product was divided into two groups: one group was subjected to enzyme digestion with methylation-sensitive restriction endonuclease Hha I, and the other group was not subjected to enzyme digestion to serve as a control. The two groups of products were respectively subjected to PCR amplification, with the addition of an Index sequence, as shown in Fig. 4A. The amplification products were subjected to hybrid capture with the µCaler hybrid Capture system, with simultaneous capture with three Panels, i.e., 10K MePanel, Normalized Panel, and OGTM800 Panel. The 10 KB probe covered a methylation-sensitive restriction endonuclease digestion site. The Normalized Panel was used for normalization. The OGTM800 Panel was used for mutation detection analysis. For the detailed experimental steps, refer to the experimental process. For the scheme for probe design, refer to the Chinese Patent Publication CN116083423A *"Probe for Targeted Enrichment of Nucleic Acid".*

The sequences of the MePanel probe are as follows.

| Probe No. SEQ ID NO: | Sequence (5' biotin) |
|---|---|
| 1 | /biotin/CGTCGGTCGGGAGAGCGGTGGGAAGTATTCGCCTCCGTTCCTCACACCGACG |
| 2 | /biotin/CGTCGGTCGACAGGATGCGAGCGCCCTTTGGAGAAGTCGCGCCACCGACG |
| 3 | /biotin/CGTCGGTCGGCGGAAACGCTAACTCCCCGGCCAAGTGCAAATGCACCGACG |
| 4 | /biotin/CGTCGGTCGGCACACACGAGGGCCCGTCGCGCCCCCCGCCCTGCCACCGACG |
| 5 | /biotin/CGTCGGTCGGCCTCGCCCTCCACGTCCCTGCACCCCCAAGTCGCACCGACG |
| 6 | /biotin/CGTCGGTCGTAAGAACCCAGTCCCCGATCGGTTTCCTCTACGCCACCGACG |
| 7 | /biotin/CGTCGGTCGGGAGGGAGGGGGGGAAAGGGGGACGAGGGGCGGACACCGACG |
| 8 | /biotin/CGTCGGTCGGGGGGGTTTGGAAGCGCACTGCCACTTGCAAAGTCCACCGACG |
| 9 | /biotin/CGTCGGTCGACCCGAGCAATCACAGCCGGAGCGCGATTCGAATGCACCGACG |
| 10 | /biotin/CGTCGGTCGTGAACCGAAGACCTCGTAGGCGGGCCTCGGGGGGCACCGACG |
| 11 | /biotin/CGTCGGTCGGATGCCGTTGGCGGCGGCCAGCGCCAGCCCCTCGGCACCGACG |
| 12 | /biotin/CGTCGGTCGGTGCCGTAGAGCAGCTGCAGCTCGCGCGCCTCGTCACCGACG |
| 13 | /biotin/CGTCGGTCGGGCTCAGGTCGCCGCAGCCCGGGAGCCTCCCCGCCACCGACG |
| 14 | /biotin/CGTCGGTCGGCCCCAAGGCACGCGCGGCACAGCCATGAACACCCACCGACG |
| 15 | /biotin/CGTCGGTCGGATGCCAAGGAGTATCTGGCCCGGAGGGAAATCCCACCGACG |
| 16 | /biotin/CGTCGGTCGGAGTCCCGCGCGCTCTCCGTGCGCCCCGGCCGGCCACCGACG |
| 17 | /biotin/CGTCGGTCGGCGGCGGCTGCCGCGCACAGGCTTCCGACTCCAGCCACCGACG |
| 18 | /biotin/CGTCGGTCGCGGCCCGCCACTGAGCATGCCCAGCACGCCGGCCCACCGACG |
| 19 | /biotin/CGTCGGTCGGAGCGGAGGGGACAGCGGGGATCGTGAGCTCCGGCCACCGACG |
| 20 | /biotin/CGTCGGTCGGGGCGAGCGGGTGCGTCTGCCGCAGAGTCGGCACCCACCGACG |
| 21 | /biotin/CGTCGGTCGAAGGACATGGAGGTAAAGGACCCCGGAGGGAGACGCACCGACG |
| 22 | /biotin/CGTCGGTCGGCACGTGCGGCGCTCAGCTTAGGCTCTCGGAGGCACACCGACG |
| 23 | /biotin/CGTCGGTCGTGAGTTGGAAATCCCGACGGAAAGCACCCACAAGCCACCGACG |
| 24 | /biotin/CGTCGGTCGCACTCTGCGCTGGCCCACCCGCGTGCACGCCCACCACCGACG |
| 25 | /biotin/CGTCGGTCGGCGGAGCTAAGCAGGGGTGTTTGGAAGGCGCCCGCACCGACG |
| 26 | /biotin/CGTCGGTCGGCTGTTTGACTGCGTGGCTTCACTTGGCCCGCGCACCGACG |
| 27 | /biotin/CGTCGGTCGATCAGCCTCTCTGGCGGGGGATTCTCCCCGGACTCCACCGACG |
| 28 | /biotin/CGTCGGTCGGGCGGGCGGGGAGCCAGGCCCGAGCTGCGTTCTGCCACCGACG |
| 29 | /biotin/CGTCGGTCGAGCCATTGGTGGGCGCCGCGCTCTGCACTGAGCACACCGACG |
| 30 | /biotin/CGTCGGTCGTCGCGCCCCGCCGGCCCCTAGCCGCAGCCGCAGCCCACCGACG |
| 31 | /biotin/CGTCGGTCGGGCAGGCGGCAGGACGGGGATCCCAGGCTGCGGGGCACCGACG |
| 32 | /biotin/CGTCGGTCGACTCCGCGGCAGAGCCGCTGGGACCTGACCCGGGACACCGACG |
| 33 | /biotin/CGTCGGTCGGCCTTCGTCCCCGCGCGCACCTCCCCGGGTCGGGCCACCGACG |
| 34 | /biotin/CGTCGGTCGAAGGGGGGCTGGTGGAATCTGGCGGTCCCCAGCCACCGACG |
| 35 | /biotin/CGTCGGTCGCGTGTCCCGGGTCGGTGCGCTCGGCGCACCCGTGCACCGACG |
| 36 | /biotin/CGTCGGTCGGACAGTGCCCGGCGTCTGCTCCCACCCGCCCGCCCCACCGACG |
| 37 | /biotin/CGTCGGTCGGAACTTGCCGGTGGTGGGGTCGTAGTGGTTTCCGACACCGACG |
| 38 | /biotin/CGTCGGTCGTTGGTGACCACGTCGTCGAACTTGAGCACCTCGTACACCGACG |
| 39 | /biotin/CGTCGGTCGTTCATGCTGCCGCTTGAGGCCGGCGTAGAAGGCGCACCGACG |
| 40 | /biotin/CGTCGGTCGGGCGGCCCGGGCACCGCGAGCCGGCCGAGCTCCACACCGACG |
| 41 | /biotin/CGTCGGTCGGGAGCTACGTGACTACGTCCACCCGCACCTACAGCACCGACG |
| 42 | /biotin/CGTCGGTCGTGGGCAGCGCGCTGCGCCCCAGCACCAGCCGCAGCCACCGACG |
| 43 | /biotin/CGTCGGTCGGGCGCCGGGCCCGCGATCGGAGCCGGAGGCACCGCCACCGACG |
| 44 | /biotin/CGTCGGTCGAGCGGAAAGAGGGCTCCGCGGCGGCCAGGCTCAGCCACCGACG |
| 45 | /biotin/CGTCGGTCGTGCCGGGAAAATGCTGGATTTAACTCCCAGTCTGCCACCGACG |
| 46 | /biotin/CGTCGGTCGTCCCGGGGATGCCAGACTCCTGGGGACGCTGGGACACCGACG |
| 47 | /biotin/CGTCGGTCGGCGGCGCGGCGGGACACGCAGGACTCCCGCCTCCACCGACG |
| 48 | /biotin/CGTCGGTCGGCCCGGAATTCGTTGAGACGGAATCTCAGCGGATCCACCGACG |
| 49 | /biotin/CGTCGGTCGTGGGGAGAAGCGCTGCGGAAAGGGGCGACTCCGACACCGACG |
| 50 | /biotin/CGTCGGTCGAGATGGCCCTGTCCCGGCGCCCCAGGTCGTCGCGCCACCGACG |
| 51 | /biotin/CGTCGGTCGGCAGCTGCGGTAGTCACTGCGCCTCCCCGCCCCCACACCGACG |
| 52 | /biotin/CGTCGGTCGCTGGATGCCCCCCTTCCCTCTCCCGGCCAGACTCCACCGACG |
| 53 | /biotin/CGTCGGTCGCGGTGCTGTGTGCCTGGCCGGAGCGTTTTGAAACACCGACG |
| 54 | /biotin/CGTCGGTCGTCCGGGGCCGCTCGGCGGCGCTGCGATTGGCCGCGCACCGACG |
| 55 | /biotin/CGTCGGTCGGGGTAACCTCTATGCAAATGAGGCCGCGCCGCCCACCGACG |
| 56 | /biotin/CGTCGGTCGGCGGGCTCGGCCCGGTCCCTCCGCTGGGGGGTTACACCGACG |
| 57 | /biotin/CGTCGGTCGAGCCGGCCGGCAGCGAGATACCCAGAGGCCCGTTCACCGACG |
| 58 | /biotin/CGTCGGTCGCACCGCGTTGCCATAGCAACGGCCTGGCCGGCTGCACCGACG |
| 59 | /biotin/CGTCGGTCGGAGGTGCCGGCTGCACAACGGCGCCCACGTGACCCCACCGACG |
| 60 | /biotin/CGTCGGTCGGTGCAGCCCCAGGCGCGCCCAAGGGCAGCTCGCCCACCGACG |
| 61 | /biotin/CGTCGGTCGGAGAAGGACCTAAAACCCCGTCTGGGGCACAGAGGCACCGACG |
| 62 | /biotin/CGTCGGTCGACATCTGGGGAAACCGAGGCGCAGAGCGGGACGGCCACCGACG |
| 63 | /biotin/CGTCGGTCGTGGGCCTGGCGGGAAGTCGCGCAAGGAGGCCGGCACACCGACG |
| 64 | /biotin/CGTCGGTCGGGGCGCACCCGGCCCACCCTGTCCCTTCCAGGCCGCACCGACG |
| 65 | /biotin/CGTCGGTCGGCTGCAGCACAGGCTGCAGGGGGCGGGCAGCGCCGCACCGACG |
| 66 | /biotin/CGTCGGTCGTCTAGCGGGGCAGGCGAGCGGAGCAGCAGCGTTCCACCGACG |
| 67 | /biotin/CGTCGGTCGAGCCCCGGCCGCGGCCCCTGAGAGGCGCGCACTCGCACCGACG |
| 68 | /biotin/CGTCGGTCGGCCTCCCTGTGCCAGCCGGCTGTCGACCCAGGTTACACCGACG |
| 69 | /biotin/CGTCGGTCGAAGCCCGAGGTCGGGGCGTTACCCCAAGGGCCCTCCACCGACG |
| 70 | /biotin/CGTCGGTCGGCTTCCCCTCCGAGGGCAGAGAGGCGTCCGCGCCCCACCGACG |
| 71 | /biotin/CGTCGGTCGGGGGCTGCGGGGTCCGTGGAACCTTTCCGGGAGGCCACCGACG |
| 72 | /biotin/CGTCGGTCGGCCGCGGGCTGCTGGGCGCGGAGGCTGGAGCCGCGCACCGACG |
| 73 | /biotin/CGTCGGTCGAGGTTCGGCTGCTGAAGCGGCCACTGCCTCCCAGACACCGACG |
| 74 | /biotin/CGTCGGTCGGATCGATGGAGAGAAGGCGGGCAAGACGCCGGGAACACCGACG |
| 75 | /biotin/CGTCGGTCGGCATTCCTCCTCAACCGAGTGCCACAACCGCCCTCCACCGACG |
| 76 | /biotin/CGTCGGTCGGAAGTGCCCCGGGGCTTCGAGCATCACCTCGCGGCACCGACG |
| 77 | /biotin/CGTCGGTCGAGCTGAGACCGGCGGCCGACGGCCAGCCCTCAGGGCACCGACG |
| 78 | /biotin/CGTCGGTCGGGTCACAAGTCAGCGCCCAAGCAAGTCAAGCGACCACCGACG |
| 79 | /biotin/CGTCGGTCGGCTCGTCTTCGCCCGAACTGATGCGCTGCAAACGCACCGACG |
| 80 | /biotin/CGTCGGTCGGGCTCAACTTCAGCGGCTTTGGCTACAGCCTGCCGCACCGACG |
| 81 | /biotin/CGTCGGTCGGAGCGCAACCGCGTCAAGTTGGTCAACCTGGGCTCACCGACG |
| 82 | /biotin/CGTCGGTCGCACCCTTCGGGAGCACGTCCCCAACGGCGCGGCCCACCGACG |
| 83 | /biotin/CGTCGGTCGAAGAAGATGAGTAAGGTGGAGACACTGCGCTCGGCACCGACG |
| 84 | /biotin/CGTCGGTCGTGGCCCTGGTTGGGTCCGCCCGCAGCGAGGGCGCACCGACG |
| 85 | /biotin/CGTCGGTCGAGACCTGCGAAGAAGTTCGGAAACTTTTCCAGTGGCACCGACG |
| 86 | /biotin/CGTCGGTCGGCTGCTGGGAGCTGTCAGGGGGCTGCCGGATTCGCCACCGACG |
| 87 | /biotin/CGTCGGTCGGCCGAGGCTGGGAGCGCGGTGATGGCCGGTCCCCGCACCGACG |
| 88 | /biotin/CGTCGGTCGAGCAGAGGGCAGCGGCGGCGGGACCGGGGACTCTGCACCGACG |
| 89 | /biotin/CGTCGGTCGCAATGGGTAAAGCCAGGAGGAAGCCCGTGCCTGGCACCGACG |
| 90 | /biotin/CGTCGGTCGAGGCGGGCAGGAGGGGAGGAGTAGGGAGGCGAGAGCACCGACG |
| 91 | /biotin/CGTCGGTCGGCGGGCGGGAGGCGCCACGGCCTCTCAGACGCTGGCACCGACG |
| 92 | /biotin/CGTCGGTCGCCGCAGCCGAGAAGCCAGAGAGAAAGTTCCCGGGCACCGACG |
| 93 | /biotin/CGTCGGTCGGCAGTAAGCGAGTAAGCGAGCCCTGGAGACCGGGCACCGACG |
| 94 | /biotin/CGTCGGTCGCTGGGTGTGCGGTCGGCTCAGTGCCACGCGGTGACACCGACG |
| 95 | /biotin/CGTCGGTCGTAGGCATGGTTATCAACCCACGCCAAGGCGGTTGCACCGACG |
| 96 | /biotin/CGTCGGTCGGCTGCCTCGCCCCGGAGCCCGGAGGAGGGGAGCCGCACCGACG |
| 97 | /biotin/CGTCGGTCGACCCGGGGAAGAAGCGGAGGACGCCGATCTGGCCCACCGACG |
| 98 | /biotin/CGTCGGTCGTGCGTTGCGCGCTCCAACCCTCTGCTTGGCCGCCCACCGACG |
| 99 | /biotin/CGTCGGTCGACAAATCCCCGAGGCGGGGAGACTTTCAGGGCATCCACCGACG |
| 100 | /biotin/CGTCGGTCGGATGCTGAAGCCTCGCGGTCCCCATTCCCAAGCCCCACCGACG |
| 101 | /biotin/CGTCGGTCGCCGTGCGCCGCCTGCGCGTGGCGCAGTTAATTTGCACCGACG |
| 102 | /biotin/CGTCGGTCGGTCTTGAGGTGGGGAGGGGAGAAATGGGAAGAGGCACCGACG |
| 103 | /biotin/CGTCGGTCGAGTAGCGGTTTTAGCCCGCTCTGCGGCTGCGAGGCACCGACG |
| 104 | /biotin/CGTCGGTCGAGATCCGAGATTAACCTCTCCCGCGATAGGTGAAGCACCGACG |
| 105 | /biotin/CGTCGGTCGTTGGGGCGACGGGGCCAGGCGCGGGGGACGGCTGCACCGACG |
| 106 | /biotin/CGTCGGTCGGGCTGGGAGGGCGCGCGGAGGAGACACCGCTGAGGCACCGACG |
| 107 | /biotin/CGTCGGTCGACGCCGCTGAGGGCGCCACGCGGGGGGCGCGGCTGCACCGACG |
| 108 | /biotin/CGTCGGTCGAGTGCGCACTTGAGGCGCGGGACGCACCGGCTGTGCACCGACG |
| 109 | /biotin/CGTCGGTCGTCCCAGGGGCTGAGACTCGCGCCCCGCCCCGCAGCCACCGACG |
| 110 | /biotin/CGTCGGTCGTCGGCTCGCCGGGCCGGGCCCCTCTCCGGAACCTCCACCGACG |
| 111 | /biotin/CGTCGGTCGGCGGCTGGAGGACGCGCTGCTGCGGATGCGCGAGGCACCGACG |
| 112 | /biotin/CGTCGGTCGTACGGGATACAGGCCGAGGAGCGGCAGGTCCGTGCCACCGACG |
| 113 | /biotin/CGTCGGTCGGGGGATGGCGCGCTGACCCCATACCCGCTGCCGTCCACCGACG |
| 114 | /biotin/CGTCGGTCGCGGCTGCGGGGCCGCCGCCGAGCGAGGGCGAGGACACCGACG |
| 115 | /biotin/CGTCGGTCGGCACCGTGCGCTTCGCCCGCAAAGGCGCCCTCCGGCACCGACG |
| 116 | /biotin/CGTCGGTCGGAAGAACGTGCATGAGGTCAAGAACCACAAATTCACACCGACG |
| 117 | /biotin/CGTCGGTCGGATGGCCGCGCCGCCCCGGGGGAGCCGGAGCCGACACCGACG |
| 118 | /biotin/CGTCGGTCGTGGCCGCCGCCCGCGCGCACTCACCAAGCCCGCTGCACCGACG |
| 119 | /biotin/CGTCGGTCGTCCTCCTCGCACGAGTACCAGATGCCGGTGTGGACACCGACG |
| 120 | /biotin/CGTCGGTCGTCCCTGGGCCGGGACTGGAGCGCAAACGGTTGGGACACCGACG |
| 121 | /biotin/CGTCGGTCGTGTCGGTGTCTGCCCGCCCGTCTGTGCGTCTGTCCACCGACG |
| 122 | /biotin/CGTCGGTCGTGTCGGTGTGAGCGTCTGAGGCGATGGGGAAGACACACCGACG |
| 123 | /biotin/CGTCGGTCGGGGCGCTTCTAGTCGGACAAAATGCAGCCGAGAACACCGACG |
| 124 | /biotin/CGTCGGTCGCGCTCGTTCTGTGCGTTCTCCTGTCCCAGGTAGGCACCGACG |
| 125 | /biotin/CGTCGGTCGAGAGGGGCTGCCGGGCGCGCTCTGCGCCCCGTTTCCACCGACG |
| 126 | /biotin/CGTCGGTCGTGGGAGCAGGGTGTCCGAAGCGCGGGTACCGATCCACCGACG |
| 127 | /biotin/CGTCGGTCGGGCAGCGCGGGGCACGCGGCGCGCGGGCGAGTCTGCACCGACG |
| 128 | /biotin/CGTCGGTCGTGCGCGCGCGGACGCCTTGGTTCCCGGAGCCCAGCACCGACG |
| 129 | /biotin/CGTCGGTCGCCACCTTCTCAGTCCGGGACGCGAGACGGGAACGCACCGACG |
| 130 | /biotin/CGTCGGTCGGCCCTGGCGCCCCAACGCTGCACCACCGTGGGTCCACCGACG |
| 131 | /biotin/CGTCGGTCGGAGCCGTGCTCGCAGTCGCTGAGATCCTCGGTGCGCACCGACG |
| 132 | /biotin/CGTCGGTCGGCGTCTCTCGCCGTCCCCTGGGCGCGGGCCAGGCGCACCGACG |
| 133 | /biotin/CGTCGGTCGGAGGAGGGGGGCGCTCCGGTCGTGTGCCCAGGACCACCGACG |
| 134 | /biotin/CGTCGGTCGCCCCAGCGGCCACTCGGGCCCCAGCCCCCCAGGCCACCGACG |
| 135 | /biotin/CGTCGGTCGTGGAGATTTAGAGCCCGGGAATGAGGCGCCGTGTCCACCGACG |
| 136 | /biotin/CGTCGGTCGTAGGAATCTCGCGCCCGGGGAGCGCTGAGGGACCGCACCGACG |
| 137 | /biotin/CGTCGGTCGGAGCCGGGTGGGGAGCGGGCCTCGGGGCGCCCGGCCACCGACG |
| 138 | /biotin/CGTCGGTCGGCAGGGCCGGAAGGAGGCCCCTGCCCCGTCCAAGGCACCGACG |
| 139 | /biotin/CGTCGGTCGGCACCAGCTGCCCCGCCGCCCGCCCGGACCCAGCCCACCGACG |
| 140 | /biotin/CGTCGGTCGGCCTCATTGCTGACGAGACCCCGCCCTGCTACTCCCACCGACG |
| 141 | /biotin/CGTCGGTCGGGCCGGAGAAGGCGCCGCTGGGGCCGCCCGGAGGACACCGACG |
| 142 | /biotin/CGTCGGTCGCCTCTGCACGGGCCCGTGGAGACGCTTCCTGCGCACCGACG |
| 143 | /biotin/CGTCGGTCGAACCTTCTGGAATCCACCGCCCCCCTTTCCGCCCGCACCGACG |
| 144 | /biotin/CGTCGGTCGCGCGCCCGCCCCCAGGAGGGCCTCCGCGAGCCGGCACCGACG |
| 145 | /biotin/CGTCGGTCGGCACACCCCGAGGCGGTCCCGGCTGCACAACTTGGCACCGACG |
| 146 | /biotin/CGTCGGTCGGAGTTGCTCCGTTTCCTCATTTTGGGGGCGAAGACACCGACG |
| 147 | /biotin/CGTCGGTCGGCCGGCGGCGGCCAGTAGCGGCGCCGTGTGCAGGGCACCGACG |
| 148 | /biotin/CGTCGGTCGAGCGCCGCCTGCGCCGCGTTCAGCACGAAGAGCTCCACCGACG |
| 149 | /biotin/CGTCGGTCGTCCAGCTCAGGCGCAGCAGCGCCACCTGGTCGGCCCACCGACG |
| 150 | /biotin/CGTCGGTCGGGCGCCCGCATTGGTGCTGACATAATTTCCTGACCCACCGACG |
| 151 | /biotin/CGTCGGTCGTGACCCGTATTGTCTCGCGATTAAAGGTAAAAAACGCACCGACG |
| 152 | /biotin/CGTCGGTCGGCTTTTTCATCCCACTGGGGTAAAACGCCCTTTTACACCGACG |
| 153 | /biotin/CGTCGGTCGAGTGGGGCCGCGGGGCCTGCTGGGAGGTGTTGTCCCACCGACG |
| 154 | /biotin/CGTCGGTCGGGAAACGTCGCTGGCGCGGAGGGATGGTTCGGCGCCACCGACG |
| 155 | /biotin/CGTCGGTCGTAGGCGTCTGTCACAGACCTATCTGCGGGTCGCCCACCGACG |

The sequences of the Normalized Pane probe are as follows.

| Probe No. SEQ ID NO: | Sequence (5' biotin) |
|---|---|
| 156 | /biotin/CGTCGGTCGCCAGCGCCATAGCCCTTAGGACTATCGGTCACACACCGACG |
| 157 | /biotin/CGTCGGTCCTCGCGCTCCTGCTCCGGCTCCTCCATCTTGGCCCACCGACG |
| 158 | /biotin/CGTCGGTCGGAGCAGAGCGAGGTGTGTGCCTCCTTACCGCCTCACCGACG |
| 159 | /biotin/CGTCGGTCGTGCACTGGACGTCCTTCCCCAGCAGCCAGTTGAGCACCGACG |
| 160 | /biotin/CGTCGGTCAGACGGAGGCTGGTGGTGCAGCAGGCAGGCAAGACCACCGACG |
| 161 | /biotin/CGTCGGTCGATGCCGGGGACTACAGCTGCGAGGCCAGGGGCCACACCGACG |
| 162 | /biotin/CGTCGGTCGCACAGCGTTGTGCATCCAGGTCTAGCGTGTCTTCCACCGACG |
| 163 | /biotin/CGTCGGTCCTGTCTTCTGTGGATGAGGTATATGAATTTATCCCAAAGCACCGACG |
| 164 | /biotin/CGTCGGTCCGAGACCTCCCAGAGCCCGTGGTTCCCTGGAGCCACACCGACG |
| 165 | /biotin/CGTCGGTCTACGAAGGGTTCCTGCTCTGTGGGCAGCTCACGAACACCGACG |
| 166 | /biotin/CGTCGGTCCAATAATGCACATGGAGAAAGTTCACCTACAATAAAGAGGCACCGACG |
| 167 | /biotin/CGTCGGTCTGTATTGGTAACATTTTATATCGAATTCCTGTTCTATGTCACCGACG |
| 168 | /biotin/CGTCGGTCAGATGTCCCCTCCCTGTCCGTCCCCGCACCTGGAGCACCGACG |
| 169 | /biotin/CGTCGGTCTGGTGGTGCATGCCCGAACCCAGCACTTCCTTGAACACCGACG |
| 170 | /biotin/CGTCGGTCCAACTGGCCAACCTAGAGCCCCCCTGGTAAAAGCCACCGACG |
| 171 | /biotin/CGTCGGTCTCTTGGTGGAATAGATGTTAATTAGTTTTTTTATTACTCCACCGACG |
| 172 | /biotin/CGTCGGTCGGAAACAATTCTGTGTTCACCCTCACCCTGCAGGCCACCGACG |
| 173 | /biotin/CGTCGGTCGGCCTCTCAGCCATCAAGACACCGTATCCTACCTCCACCGACG |
| 174 | /biotin/CGTCGGTCACGTGCTGAGCGCACGCACGTGGCGCCTGCTCACCCACCGACG |
| 175 | /biotin/CGTCGGTCGATGCTGCTCCAGCGCCCGCGCGAGTTGCGGATCGCACCGACG |
| 176 | /biotin/CGTCGGTCGTAGTTGACAAATACATTTAGTGATGTCTTATTTTTTTGGCACCGACG |
| 177 | /biotin/CGTCGGTCATCTCCTACCAGTGTATCCTTCACGACAGACGCACCACCGACG |
| 178 | /biotin/CGTCGGTCCGAGCGATGATGACACCAAATCCATGTGTCCACCCCACCGACG |
| 179 | /biotin/CGTCGGTCGGGACCCAGGAGGGCACAGCCAAGGAATGAGCCCCACCGACG |
| 180 | /biotin/CGTCGGTCCTCCTGGTCAGCAGCCTCTTAGAGAACCTGCTGGACACCGACG |
| 181 | /biotin/CGTCGGTCTATAGAACCATCATCATGCAAGATGAGAGCAAGGAGCACCGACG |
| 182 | /biotin/CGTCGGTCGAACAGTAAACAGTGGTTCTGACTGGTGAAATGATAGTTACACCGACG |
| 183 | /biotin/CGTCGGTCGTTGAAAGGACCAGCACTTTATTTATTGCTTTAGCCATTCACCGACG |
| 184 | /biotin/CGTCGGTCAGCCTGCACGGGAGTCAGAGGCACTCAGCAATGCCCACCGACG |
| 185 | /biotin/CGTCGGTCTGGCTCTGTTACGAACGGCTGAAATCAAAACCCCCACCGACG |
| 186 | /biotin/CGTCGGTCTCACCGTGTTCTCCAGACCATGCATGTTCCTCCGCCACCGACG |
| 187 | /biotin/CGTCGGTCACCGCCCCTTCAGGGATGGGGCTGTAGGAGTCAAGCACCGACG |
| 188 | /biotin/CGTCGGTCTCTCGGTGTCTCCCCAGGTGCAAGTGCAACCTGCACACCGACG |
| 189 | /biotin/CGTCGGTCGCCAACCTGTGCTCCATGCGCGAGGGCAGCCTGCACACCGACG |
| 190 | /biotin/CGTCGGTCAAGCCCACTCTTCTACTCCCCGAACGTGCCTTCCCCACCGACG |
| 191 | /biotin/CGTCGGTCCTCTGACGGCGACAGTAGCTCCGTGGACAGCGATGCACCGACG |
| 192 | /biotin/CGTCGGTCCTGGAGGAGCACGGAAAAGACCTGGAAATCATGCACACCGACG |
| 193 | /biotin/CGTCGGTCATCCTCACCAGGGTGAATGACAGAGTTGCCAGGCACACCGACG |
| 194 | /biotin/CGTCGGTCGCATAGGAGTAGGCCAGCCAGCGGAACACGTGGTCCACCGACG |
| 195 | /biotin/CGTCGGTCTCGGGGGTGGGGGTGTGTTCCTGCGTCTTCCAGGGCACCGACG |
| 196 | /biotin/CGTCGGTCCGGGAATGCCGAGTAAGTGTTAATTTTATGGTCCCCACCGACG |
| 197 | /biotin/CGTCGGTCATCACTTCTGGAAAATGAATAGATAGATGTGTGGGCTTTACACCGACG |
| 198 | /biotin/CGTCGGTCGTGCCTCAATGATCTTTTTTCCCCACCTTCATTTTTCTCACCGACG |
| 199 | /biotin/CGTCGGTCCTTCAGCGGCATCTGCTAGCTCGTCTACCCCTGTCACCGACG |
| 200 | /biotin/CGTCGGTCCATGTTTTAGGGAACTCTGCCCTATAAACACTCATAGACACCGACG |
| 201 | /biotin/CGTCGGTCGGTTTCTCTCTATGTTGCAGTCCCTCTGTCGTGAACACCGACG |
| 202 | /biotin/CGTCGGTCCGGCATTCCTGTGGTAGTGGCCTGAGAACACGACCACCGACG |
| 203 | /biotin/CGTCGGTCTGACACCTGCAGAGAAGGGAAAAAGTCATTAGGGGCACCGACG |
| 204 | /biotin/CGTCGGTCACATGTCCCTGCGTCACAGGCACCTGCAGAGCCCCCACCGACG |
| 205 | /biotin/CGTCGGTCTGTGTAAGGCGTTCCGGCACGTCAAGGTGGACACACACCGACG |
| 206 | /biotin/CGTCGGTCTGAAGATACAAACAACCACAGCAGCCTGCACTTACCACCGACG |
| 207 | /biotin/CGTCGGTCGTGTGGTCATCTTCTTCTTCCCCTTCTAGCACGACCACCGACG |
| 208 | /biotin/CGTCGGTCACGTTGCATCGACCCTCTCCACTCCGCGGAAGTAGCACCGACG |
| 209 | /biotin/CGTCGGTCCGACACCGTCTGATGTGTTGCGCAGGTGGTGGCACACCGACG |
| 210 | /biotin/CGTCGGTCCATTGGGATGGAGGGTGGGGAAGGAGTCACCCTGGCACCGACG |
| 211 | /biotin/CGTCGGTCCCTCCTCCGCCTGACTTACTTGATCCTAAAGTCACACCGACG |
| 212 | /biotin/CGTCGGTCCTCCCAGGGCTCCTTGGCCTCCTCCGCGGTGACCCACCGACG |
| 213 | /biotin/CGTCGGTCGGGACACAGGGCCGCATGAGCCTGGGCGGGGTCAGCACCGACG |
| 214 | /biotin/CGTCGGTCCGCCCTCCGAGGAGGGCCCGGGCGGGGTGGGCGCCCACCGACG |
| 215 | /biotin/CGTCGGTCCGTCCTCAGGCTCCAGGCTGGGAGGAGAGAGCGTCACCGACG |
| 216 | /biotin/CGTCGGTCGCTGGGAGTGGAGCTGGGGGTCAGAAGAGCAAGCACACCGACG |
| 217 | /biotin/CGTCGGTCAACGAAATTGATCAAACTTAGACTGCTTCACCTGTCTCCACCGACG |
| 218 | /biotin/CGTCGGTCGCGTGGACAGGCAGCGCCGCAGTGACGTCTCCATGCACCGACG |
| 219 | /biotin/CGTCGGTCTGGAGTCCTCCAGCGGCTTCAGGCGGTTCAGCTTCCACCGACG |

Result analysis: The OGTM-CK experimental group serves as the control and is not subjected to enzyme digestion with the methylation-sensitive restriction endonuclease. The samples in this group can be used for analysis of mutation detection. The OGTM-MSRE group is the enzyme digestion group and is subjected to enzyme digestion with the methylation-sensitive restriction endonuclease. The OGTM800 Panel designed in this process does not involve the methylation-sensitive restriction endonuclease digestion site. The samples in this group can be used for analysis of methylation and mutation detection. Fig. 4B shows the on-target rate and the mappability, with the mappability greater than 99%. Fig. 4C shows the covered depth of different experimental groups, where 0.2× Mean Coverage > 95% and 0.5× Mean Coverage > 70%. Fig. 5 shows the sequencing depths and corresponding methylation levels of CpG sites associated with a methylation-sensitive restriction endonuclease within target regions of different libraries, where a methylation level calculation method: enzyme digestion depth/control depth; (A) Mean CpG methylation level of two enzyme digestion experimental groups; (B) Methylation level corresponding to each CpG site. Fig. 6 shows the consistency analysis between detected mutation frequencies and theoretical mutation frequencies, where the designed probes cover 15 mutation sites, including 11 mutation sites, 2 fusion sites, and 2 copy number variations (base insertions and deletions), involving common mutation detection variation sites of cancers. The corresponding mutation frequencies range from 1% to 7%. The OGTM-CK experimental group is not subjected to enzyme digestion. The OGTM-MSRE experimental group is subjected to enzyme digestion with a methylation-sensitive restriction endonuclease. All the mutation Panel coverage sites selected in this example do not include an endonuclease recognition site, so the detection results of both the OGTM-CK and OGTM-MSRE groups can reflect the true mutation levels of the samples. The results of the consistency analysis between detected mutation frequencies and theoretical mutation frequencies indicate that all the results detected from different experimental groups align with theoretical expectations. OGTM-CK represents the mutation detection conditions of the control group in Fig. 2A. If the mutation detection site includes a methylation-sensitive restriction endonuclease digestion site, it is recommended to adopt this experimental design scheme. OGTM-MSRE represents the mutation detection results in Fig. 2B. If the mutation detection site does not involve a methylation-sensitive restriction endonuclease digestion site, it is recommended to adopt this experimental design scheme.

## Claims

1. A method for simultaneously detecting a methylation state and a mutation state of a DNA molecule in a sample, comprising:
1) adding adapter molecules to both ends of the DNA molecule in the sample, thereby obtaining an adapter ligation product sample; and dividing the adapter ligation product sample into two parts: a first adapter ligation product sample and a second adapter ligation product sample;
2) treating the first adapter ligation product sample with one or more methylation-sensitive restriction endonucleases, so that when the DNA molecule lacks methylation at a recognition site of the methylation-sensitive restriction endonuclease, proceeding an enzyme cutting reaction , thereby obtaining an enzyme digestion sample; and untreating the second adapter ligation product sample with the restriction endonuclease;
3) amplifying the enzyme digestion sample and the second adapter ligation product sample respectively with a primer specific to the adaptor molecule and having an Index sequence, thereby obtaining an enzyme digestion sample amplification product and a second adapter ligation product sample amplification product, respectively; and mixing the enzyme digestion sample amplification product with the second adapter ligation product sample amplification product and obtaining an amplification product mixture;
4) making a probe group comprising one or more mutation capture probes and one or more methylation capture probes in contact with the amplification product mixture, thereby obtaining a capture product, wherein the methylation capture probes target a target sequence comprising the recognition site in the DNA molecule, and the mutation capture probes target a target sequence comprising a mutation site in the DNA molecule; and
5) sequencing the capture product; determining the methylation state of the DNA molecule comprising the recognition site through a sequencing depth ratio of the recognition sites from the enzyme digestion sample amplification product to the second adapter ligation product sample amplification product; and determining the mutation state through a sequencing result of the mutation site.

2. The method as claimed in claim 1, wherein the mutation capture probe and/or the methylation capture probe comprise a target specific sequence, a first probe binding sequence located at a 5' end of the target specific sequence, and a second probe binding sequence located at a 3' end of the target specific sequence; and the first probe binding sequence is at least partially complementary to the second probe binding sequence, so that when two or more of the mutation capture probes and/or two or more of the methylation capture probes bind to a target sequence thereof adjacently, the adjacent mutation capture probes and/or the adjacent methylation capture probes are capable of being complementarily bound through the first probe binding sequence and the second probe binding sequence.

3. The method as claimed in claim 1 or 2, wherein the probe group further comprises one or more normalization probes.

4. The method as claimed in any one of claims 1-3, wherein the first probe binding sequence and the second probe binding sequence have a length of 8-30 nt.

5. The method as claimed in any one of claims 1-4, wherein the target specific sequence has a length of 20-80 nt.

6. The method as claimed in any one of claims 1-5, prior to step 1), further comprising, fragmenting the DNA molecule.

7. The method as claimed in any one of claims 1-6, wherein the methylation-sensitive restriction endonuclease is selected from Hpa I, Hpa II, Hha I, Aci I, and any combination thereof.

8. The method as claimed in any one of claims 1-7, wherein the sample comprises a ctDNA.

9. The method as claimed in any one of claims 1-8, wherein the methylation state comprises whether methylation is present and/or a methylation level.

10. The method as claimed in any one of claims 1-9, wherein the mutation state comprises whether mutation is present and/or a mutation type.

11. The method as claimed in any one of claims 1-10, wherein the mutation type is selected from base insertion, deletion and substitution, chromosomal copy number variation, microsatellite instability, and gene fusion.
